(19) Europäisches Patentamt
European Patent Office
Office européen des brevets

(11) **EP 4 632 078 A2**

(12) **EUROPEAN PATENT APPLICATION**

(43) Date of publication:
**15.10.2025 Bulletin 2025/42**

(21) Application number: **25196945.7**

(22) Date of filing: **22.01.2020**

(51) International Patent Classification (IPC):
***C12Q 1/6881*** *(2018.01)*

(52) Cooperative Patent Classification (CPC):
(C-Sets available)
**C12Q 1/6809; C12Q 1/6881** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.01.2019 US 201962796481 P**

(62) Document number(s) of the earlier application(s) in
accordance with Art. 76 EPC:
**20708291.8 / 3 914 731**

(71) Applicant: **Illumina, Inc.
San Diego, CA 92122 (US)**

(72) Inventor: **LI, Yong
San Diego, CA 92122 (US)**

(74) Representative: **Witte, Weller & Partner
Patentanwälte mbB
Postfach 10 54 62
70047 Stuttgart (DE)**

Remarks:
This application was filed on 20.08.2025 as a
divisional application to the application mentioned
under INID code 62.

(54) **METHODS AND SYSTEMS FOR MONITORING ORGAN HEALTH AND DISEASE**

(57) Methods, compositions, and systems are provided for monitoring tissue and organ health. The methods, compositions, and systems provided herein extract locus specific copy number signals from cell free DNA (cfDNA) samples to identify tissue-specific cfDNA copy number profiles and enable quantification of tissue fractions in the cfDNA samples.

(52) Cooperative Patent Classification (CPC): (Cont.)

C-Sets
**C12Q 1/6809, C12Q 2535/122, C12Q 2537/16;**
**C12Q 1/6881, C12Q 2535/122, C12Q 2537/16**

**Description**

FIELD

Systems, methods, and compositions provided herein relate to methods for extracting locus-specific cfDNA copy number signals from a sample for health monitoring, diagnostics, or cellular profiling and analysis. Specifically, the systems, methods, and compositions relate to methods for analyzing cell free DNA (cfDNA) in a sample to determine a relative contribution of tissue or cell type to total cfDNA in a sample. Methods provided herein utilize the sequence specific cfDNA coverage, intensity, or copy number signals and does not involve direct determination of methylation status on cfDNA.

BACKGROUND

In recent years, cell free DNA (cfDNA) has emerged as a promising source for biomarker discovery for disease diagnostics. In particular, fetal cfDNA and intact fetal cells can enter maternal blood circulation. Consequently, analysis of this fetal genetic material can allow early non-invasive prenatal testing (NIPT). A key challenge in performing NIPT on fetal cfDNA is that it is typically mixed with maternal cfDNA, and thus the analysis of the cfDNA is hindered by the need to account for the maternal genotypic signal. Furthermore, analysis of cfDNA is useful as a diagnostic tool for detection and diagnosis of cancer.

Current protocols for preparing a sequencing library from a cell-free nucleic acid sample (e.g., a plasma sample) typically involve isolating cfDNA for preparation of a sequencing library for analysis. However, existing methods of analyzing cfDNA, whether for NIPT or oncology applications, rely on extracting a signal of genetic changes from cfDNA sequencing, and are therefore limited to NIPT and oncology.

SUMMARY

The present disclosure relates to systems, methods, and compositions for analyzing cfDNA in a sample to extract cfDNA locus-specific copy number signals for quantifying tissue and/or cell specific fractions of cfDNA in the sample.

Some embodiments provided herein relate to methods of analyzing cell free DNA (cfDNA) in a biological sample. In some embodiments, the sample is from a human subject with potential cell death, or tissue or disease damage. In some embodiments, cell death or tissue/organ damage include blunt trauma, such as head trauma, drug toxicity on liver or kidney, diseases that involve organ damage, such as heart damage in cardiomyopathies, kidney damage in kidney diseases, liver damage in liver diseases, or beta cell death in diabetes. In some embodiments, cell death or tissue/organ damage include cancer or pregnancy, for which excessive amounts of cell death or cell turn-over occurs.

In some embodiments, the methods include obtaining a biological sample comprising cfDNA, wherein the cfDNA comprises a plurality of cfDNA fragments, each fragment corresponding to one or more tissues or cell types; quantifying each cfDNA fragment to generate a genome-wide or targeted (locus specific) cfDNA profile, wherein the genome-wide cfDNA profile comprises a plurality of copy number signals, each copy number (including coverage or intensity) signal corresponding to a cfDNA fragment; and comparing the genome-wide cfDNA copy number signal profile to a collection of reference copy number signal profiles to determine or quantify sources of cell damage, tissue damage, or organ damage. In some embodiments, the method optionally includes enriching cfDNA through pull down or PCR from the sample to provide enriched cfDNA.

Some embodiments provided herein relate to methods of monitoring the progress of tissue or organ damage in a subject. In some embodiments, the methods include obtaining a biological sample from the subject, wherein the biological sample comprises cell free DNA (cfDNA); quantifying the cfDNA in the sample to obtain a genome-wide cfDNA copy number signal profile comprising a plurality of copy number signals, each copy number signal corresponding to a cfDNA fragment of a specific cell type or tissue type; and comparing the genome-wide cfDNA copy number signal profile to a collection of known copy number signal profiles of healthy subjects or pure tissue types. In some embodiments, the quantifying is performed without PCR or enrichment. In some embodiments, a difference of copy number signal in the sample compared to the known copy number signals correlates to a condition in the subject related to tissue or organ damage.

BRIEF DESCRIPTION OF THE DRAWINGS

FIG. 1 illustrates a plot depicting kidney tissue and blood signal profiles of cfDNA along targeted chromosome locations. The tissue/cell type specific signal is extracted using non-negative matrix factorization methods from kidney disease patients' plasma cfDNA copy number signals obtained from cfDNA sequencing. The target regions are assayed through multiplex PCR on cfDNA samples.

FIG. 2 depicts a plot showing results for predicting kidney failure in patients based on quantifications of the fraction of kidney cfDNA in blood plasma.

FIGs. 3A and 3B depict plots for time course pattern of the proportion of DNA from kidney tissue as a function of time in a set of kidney transplant recipients. FIG. 3A shows the estimated kidney fraction of donor kidney cfDNA, and FIG. 3B shows the estimated kidney fraction of the patient's own kidney cfDNA. Both FIGs. 3A and 3B show statistically significant changes over time, and the pattern of temporal changes is consistent with biomedical procedures known for these patients.

FIG. 4 depicts the component fraction of colon cfDNA across various diseases, where the fraction for Crohn's disease was found to be significantly greater than in other diseases analyzed.

FIG. 5 depicts a block diagram illustrating a process for evaluating cfDNA samples for tissue cfDNA quantification.

DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein.

Embodiments of the systems, methods, and compositions provided herein relate to analyzing nucleic acid fragments in a sample to determine how many nucleic acid fragments originate from various parts of the genome of various parts of a body of a subject. More particularly, the systems, methods, and compositions provided herein relate to analyzing cfDNA populations in a sample to determine a relative amount of cfDNA from various parts of a genome of various parts of a body of a subject. The systems, methods, and compositions therefore relate to tissue origin quantification of cfDNA and may be used in broad applications involving elevated cell death or elevated genetic alterations, including, for example, for monitoring disease progression, monitoring organ or tissue health, diagnosing or detecting disease, determining drug efficacy or toxicity, or newborn health monitoring.

In one embodiment, a biological sample that is known to carry cfDNA, such as blood plasma, is taken from a subject suspected of having a specific type of organ damage or elevated cell turn over. A whole genome sequence (WGS) analysis is performed on the cfDNA in the biological sample to identify genomic regions that may show more or less cfDNA than in a typical subject. For example, if the subject suffers from liver damage or kidney failure, one may expect to see more cfDNA derived from the liver or kidney as compared to a baseline control population. Once the sequence analysis is completed, it is compared through a variety of different machine learning, artificial intelligence, or other protocols to identify differences in the cfDNA from the subject to a baseline control. In one embodiment, part of the analysis may include quantifying the relative fractions of cfDNA from different tissues from the subject and normal baseline controls. In some embodiments, quantification may include one or both of determining the set of reference tissue profiles, and quantifying the fractions of tissue cfDNA in a cfDNA sample based upon a genome-wide cfDNA coverage data.

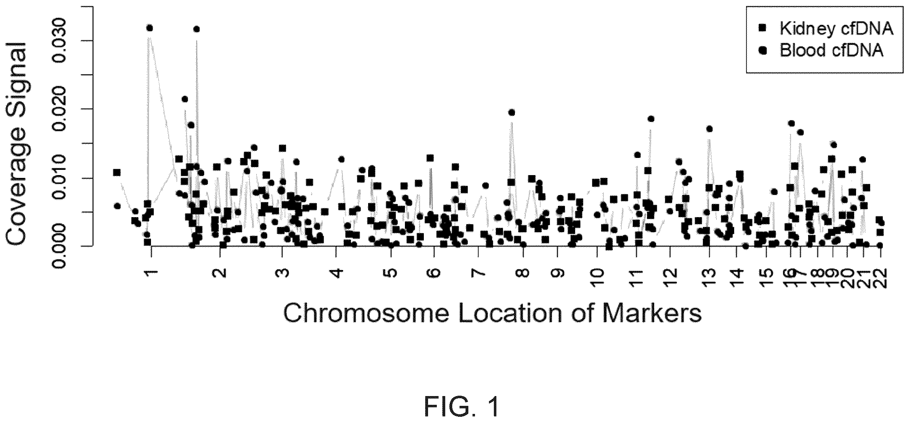

For example, for a genome wide or targeted cfDNA copy number profiles for a set of normal and/or diseased samples, a set of reference cfDNA coverage profiles are derived and the resulting linear combination reconstructs the cfDNA copy number signals from normal and/or diseased samples. Each reference profile corresponds to a specific cell or tissue type. Using unsupervised machine learning methods such as non-negative matrix factorization, cfDNA signals from individuals may be decomposed and the reference tissue or cell specific profiles extracted, thereby generating baseline reference profiles. Depending on the body fluid type, the dominant cell or tissue types may be different. For example, for plasma, white blood cell signal profiles would be the major contributors. An exemplary analysis of extracted kidney tissue and blood signal profiles of cfDNA along targeted chromosome locations is depicted in Figure 1.

Traditional methods of analyzing cfDNA require sequence specific detection, which limits the sensitivity of the assay and does not provide accurate, reliable, or reproducible determinations of a relative contribution of each tissue type in the subject to the total cfDNA in a biological sample. For example, the traditional approach may not determine how much of the cfDNA in the sample came from lung, spleen, liver, kidney, etc. as compared to a normal sample. Prior methods of cfDNA sequencing were for applications relating to monitoring the status of transplant tissues or cancers. However, such methods require an allele-based analysis, which required sequencing and detection of single nucleotide variations between donor and host or tumor and normal. There is no existing method that can quantify a subject's own organ health status from cfDNA sequencing, array hybridization, or similar methods.

Further, traditional methods for monitoring organ or tissue health are performed through tissue biopsy. Tissue biopsy may be used to examine and determine a presence or extent of a disease based on a specific tissue, and may be performed by extraction of cells or tissue from a tissue biopsy sample taken from a subject. However, these methods are invasive, time-consuming, expensive, and generally carry increased risks of unintended health consequences.

The systems, methods, and compositions described herein, in contrast, relate to determining a quantity of cfDNA fragments that originate from various tissues. Furthermore, the present systems, methods, and compositions are non-invasive and can provide an immediate determination of the dynamics of cell death or tissue damage. The systems, methods, and compositions provided herein may allow for early detection of a variety of indications before clinical symptoms or functional deterioration of a subject's body is found. Moreover, these methods do not require selection of a specifically targeted organ, but instead enable a care-giver to discover which organ may be deteriorating, which is not possible using tissue biopsy as a screening method. Relatedly, the methods, systems, and compositions can enable quantification and monitoring of multiple organs at once, in a single analysis, with less sampling bias than tissue biopsy methods.

Unless otherwise indicated, the practice of the method and system disclosed herein involves conventional techniques and apparatus commonly used in molecular biology, microbiology, protein purification, protein engineering, protein and DNA sequencing, and recombinant DNA fields, which are within the skill of the art. Such techniques and apparatus are known to those of skill in the art and are described in numerous texts and reference works (See e.g., Sambrook et al., "Molecular Cloning: A Laboratory Manual," Third Edition (Cold Spring Harbor), [2001]); and Ausubel et al., "Current Protocols in Molecular Biology" [1987]).

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless defined otherwise herein, all technical and scientific terms used herein have the same meaning as commonly understood by one of ordinary skill in the art. Various scientific dictionaries that include the terms included herein are well known and available to those in the art. Although any methods and materials similar or equivalent to those described herein find use in the practice or testing of the embodiments disclosed herein, some methods and materials are described.

The terms defined immediately below are more fully described by reference to the Specification as a whole. It is to be understood that this disclosure is not limited to the particular methodology, protocols, and reagents described, as these may vary, depending upon the context they are used by those of skill in the art. As used herein, the singular terms "a," "an," and "the" include the plural reference unless the context clearly indicates otherwise.

Unless otherwise indicated, nucleic acids are written left to right in 5' to 3' orientation and amino acid sequences are written left to right in amino to carboxy orientation, respectively.

As used herein "polynucleotide" and "nucleic acid", may be used interchangeably, and can refer to a polymeric form of nucleotides of any length, either ribonucleotides or deoxyribonucleotides. Thus, these terms include single-, double-, or multi-stranded DNA or RNA. Examples of polynucleotides include a gene or gene fragment, cell free DNA (cfDNA), whole genomic DNA, genomic DNA, epigenomic, genomic DNA fragment, exon, intron, messenger RNA (mRNA), regulatory RNA, transfer RNA, ribosomal RNA, non-coding RNA (ncRNA) such as PIWI-interacting RNA (piRNA), small interfering RNA (siRNA), and long non-coding RNA (lncRNA), small hairpin (shRNA), small nuclear RNA (snRNA), micro RNA (miRNA), small nucleolar RNA (snoRNA) and viral RNA, ribozyme, cDNA, recombinant polynucleotide, branched polynucleotide, plasmid, vector, isolated DNA of any sequence, isolated RNA of any sequence, nucleic acid probe, primer or amplified copy of any of the foregoing. A polynucleotide can include modified nucleotides, such as methylated nucleotides and nucleotide analogs including nucleotides with non-natural bases, nucleotides with modified natural bases such as aza- or deaza-purines. A polynucleotide can be composed of a specific sequence of four nucleotide bases: adenine (A); cytosine (C); guanine (G); and thymine (T). Uracil (U) can also be present, for example, as a natural replacement for thymine when the polynucleotide is RNA. Uracil can also be used in DNA. The term "nucleic acid sequence" can refer to the alphabetical representation of a polynucleotide or any nucleic acid molecule, including natural and non-natural bases.

The term donor DNA (dDNA) refers to DNA molecules originating from cells of a donor of a transplant. In various implementations, the dDNA is found in a sample obtained from a donee who received a transplanted tissue or organ from the donor.

Circulating cell-free DNA or simply cell-free DNA (cfDNA) are DNA fragments that are not confined within cells and are freely circulating in the bloodstream or other bodily fluids. It is known that cfDNA have different origins, in some cases from donor tissue DNA circulating in a donee's blood, in some cases from tumor cells or tumor affected cells, in other cases from fetal DNA circulating in maternal blood. Other non-limiting examples include cfDNA originating from tissue or organs native to the same organism, such as kidney, lung, brain, and heart, for example. Levels of tissue-specific cfDNA may increase or decrease where cell death, tissue damage or organ damage occurs, including for example, blunt trauma such as head trauma, drug toxicity in liver or kidney, diseases that involved organ damage such as heart damage in cardiomyopathies, kidney damage in kidney disease, liver damage in liver disease, and beta cell death in diabetes. Examples also include cancer and pregnancy, for which excessive amount of cell death or cell turnover occurs.

In general, cfDNA are fragmented and include only a small portion of a genome, which may be different from the genome of the individual from which the cfDNA is obtained. The exact mechanism of cfDNA biogenesis is unknown. It is generally believed that cfDNA comes from apoptotic or necrotic cell death, however there are also evidences suggesting active cfDNA release from living cells. Generally, cfDNA originates from diverse cell types, and depending on the cell origin and the health status, the genome wide cfDNA profile of a subject may vary.

The term non-circulating genomic DNA (gDNA) or cellular DNA are used to refer to DNA molecules that are confined in cells and often include a complete genome.

A binomial distribution is a discrete probability distribution of the number of successes in a sequence of n independent experiments, each asking a yes-no question, and each with its own Boolean-valued outcome: a random variable containing single bit of information: positive (with probability p) or negative (with probability q = 1 - p). For a single trial, i.e., n = 1, the binomial distribution is a Bernoulli distribution. The binomial distribution is frequently used to model the number of successes in a sample of size n drawn with replacement from a population of size N. If the random variable $X$ follows the binomial distribution with parameters $n \in \mathbb{N}$ and $p \in [0,1]$, the random variable $X$ is written as $X \sim \mathrm{B}(n, p)$.

Poisson distribution, denoted as Pois() herein, is a discrete probability distribution that expresses the probability of a given number of events occurring in a fixed interval of time and/or space if these events occur with a known average rate and independently of the time since the last event. The Poisson distribution can also be used for the number of events in other specified intervals such as distance, area or volume. The probability of observing k events in an interval according to a Poisson distribution is given by the equation:

$$P(k \text{ events in interval}) = \frac{\lambda^k e^{-\lambda}}{k!}$$

where $\lambda$ is the average number of events in an interval or an event rate, also called the rate parameter e is 2.71828, Euler's number, or the base of the natural logarithms, k takes values 0, 1, 2, ..., and $k!$ is the factorial of $k$.

Gamma distribution is a two-parameter family of continuous probability distributions. There are three different parametrizations in common use: with a shape parameter k and a scale parameter $\theta$; with a shape parameter $\alpha$ = k and an inverse scale parameter $\beta = 1/\theta$, called a rate parameter; or with a shape parameter k and a mean parameter $\mu = k/\beta$. In each of these three forms, both parameters are positive real numbers. The gamma distribution is the maximum entropy probability distribution for a random variable X for which $E[X] = k\theta = \alpha/\beta$ is fixed and greater than zero, and $E[\ln(X)] = \psi(k) + \ln(\theta) = \psi(\alpha) - \ln(\beta)$ is fixed ($\psi$ is the digamma function).

The term "sample" herein refers to a sample typically derived from a biological fluid, cell, tissue, organ, or organism, comprising a nucleic acid or a mixture of nucleic acids, and may be referred to herein as a biological sample. Such samples include, but are not limited to sputum/oral fluid, amniotic fluid, blood, a blood fraction, or fine needle biopsy samples (e.g., surgical biopsy, fine needle biopsy, etc.), urine, peritoneal fluid, pleural fluid, and the like. Although the sample is often taken from a human subject (e.g., patient), the assays can be used in samples from any mammal, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc. If such methods of pretreatment are employed with respect to the sample, such pretreatment methods are typically such that the nucleic acid(s) of interest remain in the test sample, sometimes at a concentration proportional to that in an untreated test sample (e.g., namely, a sample that is not subjected to any such pretreatment method(s)). Such "treated" or "processed" samples are still considered to be biological "test" samples with respect to the methods described herein.

The term "biological fluid" herein refers to a liquid taken from a biological source and includes, for example, blood, serum, plasma, sputum, lavage fluid, cerebrospinal fluid, urine, semen, sweat, tears, saliva, and the like. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The sample may be obtained from a subject, wherein it is desirable to monitor tissue or organ health, diagnose or detect a disease, or otherwise analyze a sample of a subject. As used herein, a "subject" refers to an animal that is the object of treatment, observation, or experiment. "Animal" includes cold- and warm-blooded vertebrates and invertebrates such as fish, shellfish, reptiles and, in particular, mammals. "Mammal" includes, without limitation, mice, rats, rabbits, guinea pigs, dogs, cats, sheep, goats, cows, horses, primates, such as monkeys, chimpanzees, and apes, and, in particular, humans. The subject may be a subject having or suspected of having cancer, a genetic disorder, organ damage or tissue damage, or other disease or disorder that can be monitored. In some embodiments, the subject is an organ donee, such as a subject that is the recipient of an organ transplant. In some embodiments, the subject has potential organ damage due to a chronic illness or blunt trauma.

Embodiments of the systems, methods, and compositions relate to obtaining a sample from a subject and monitoring, detecting, evaluating, predicting, or diagnosing a disease or disorder in the subject, monitoring tissue or organ damage in a subject, or evaluating or quantifying nucleic acid tissue origin. Diseases may include, for example, cancers, genetic disorders, organ specific disorders, or other diseases or disorders that are characterized by increased cfDNA in different genomic regions based on tissue origin and/or disease type.

As used herein, the term reference genome refers to any particular known genome sequence, whether partial or complete, of any organism that may be used to reference identified sequences from a subject. A "genome" refers to the complete genetic information of an organism or virus, expressed in nucleic acid sequences.

Some embodiments of the methods, systems, and compositions provided herein relate to simultaneously quantifying relative contributions of multiple tissues or cell types in a cfDNA sample, based on genome wide cfDNA copy number (CN) signals. Depending on the intended application, the cfDNA sample can be derived from a biological sample, for example, from blood, plasma, urine, cerebrospinal fluid, or any other types of human body fluid. The genome wide cfDNA coverage, copy number, or intensity signals can be obtained through sequencing-based DNA molecule counting, such as by any sequencing technologies, or by hybridization-based DNA copy number quantification technologies. In some embodiments, the cfDNA may be subjected to targeted PCR or an enrichment assay or genome wide amplifications prior to copy number signal measurements. In any of the embodiments, various amplification methods may be used, including, for example non-specific amplification of the entire genome, for example, whole genome amplification (WGA) methods such as MDA, or highly targeted PCR amplification of a few or a single selected region of, for example, a few kb.

Given the cfDNA coverage from a biological sample or a set of biological samples from any of the systems or methods described herein, relative fractions of different tissues may be quantified. In some embodiments, quantification may include one or both of determining the set of reference tissue profiles, and quantifying a fraction of tissue cfDNA in a cfDNA sample based upon a genome-wide or targeted cfDNA coverage data.

For example, for genome wide cfDNA copy number profiles for a set of normal samples, a set of reference cfDNA coverage profiles are derived such that the resulting linear combinations correspond to the cfDNA copy number profiles from the normal samples. Whereas a blood cfDNA copy number profile corresponds to a mixture of signals from multiple cell or tissue types, a reference profile corresponds to a specific cell or tissue type. Using unsupervised machine learning methods such as non-negative matrix factorization, a set of plasma cfDNA signals may be decomposed and the reference profiles extracted, thereby generating a set of baseline reference profiles. Depending on the body fluid type, the dominant cell or tissue types may be different. For example, for plasma white blood cell, signal profiles would be the major contributors.

Similarly, from the genome wide cfDNA copy number profiles for a set of patient samples with known organ damage or a specific disease associated with organ damage, semi-supervised machine learning may be employed to extract the tissue or disease specific cfDNA profiles in addition to the baseline reference profiles. The baseline reference profiles obtained may be used to account for the baseline portion of the cfDNA signal from the patient samples, and additional tissue reference profiles are then derived from the unaccounted cfDNA coverage signals.

The unsupervised and semi-supervised approach may be further coupled with a supervised machine learning method based on deep neural network to predicted cfDNA coverage profiles for tissue or cell types for which access to relevant cfDNA samples are limited. The deep learning method may be used to predict cfDNA coverage profile for a cell type given the epigenetic signals for the given cell type as input features, including, for example, DNase accessibility signals, histone mark signals, and genomic DNA methylation signals.

Accordingly, in some embodiments, a set of reference tissue profiles are used for tissue quantification on samples of interest. For a cfDNA coverage profile, the tissue fractions may be quantified by linearly projecting the observed cfDNA coverage profiles onto the known reference profiles.

Embodiments of the systems, methods, and compositions provided herein may include broad applications, including, for example, organ health monitoring, drug toxicity monitoring, sports medicine, disease diagnosis and detection, oncology, non-invasive prenatal testing (NIPT) and newborn health monitoring, or disease pathology research.

In the field of organ health monitoring, embodiments of the systems, methods, and compositions may be used, for example, for monitoring multiple organs, such as, for example, the kidney, lung, or heart, and for pre- and post-disease monitoring and diagnosis from a single blood test. The embodiments described herein include a low cost universal blood test targeting the major organs, enabling early detection and prevention of severe organ failures, including for monitoring strategy for high-risk populations. For example, kidney health monitoring for patients having lupus or diabetes; heart health monitoring for individuals with family history of cardiomyopathy; or multiple-organ health monitoring for patients with sepsis. Furthermore, the severity of trauma (blunt injury), for example, on head or chest/lung region, are not easy to access unless severe functional consequence is observed. Embodiments of the systems, methods, and compositions provided herein enable quantitative monitoring of the severity of trauma, and inform early medical interventions.

In the field of drug toxicity monitoring, embodiments of the systems, methods, and compositions may be used, for example, for monitoring liver or renal toxicity of a prescription drug in a given patient, thereby enabling personalized medicine and real-time adjustment to medication regimens for individual patients, or measuring the liver or renal drug toxicity of new drugs in clinical trials.

In the field of sports medicine, embodiments of the systems, methods, and compositions may be used, for example, for monitoring the magnitude of body damage due to intense training, thereby enabling rational tuning of athlete training schedule and preventing over training syndrome. Cell free DNA is found to increase with exercise. For athletes, over training syndrome (OTS) is a frequent occurring condition when they constant push for the limit. Once OTS occurs, it can take days to weeks to recover, or in some cases, the athletes may never recover. An approach for muscle cfDNA quantification, and hence early detection and prevention of OTS would be of high value for athlete to achieve optimal training outcome.

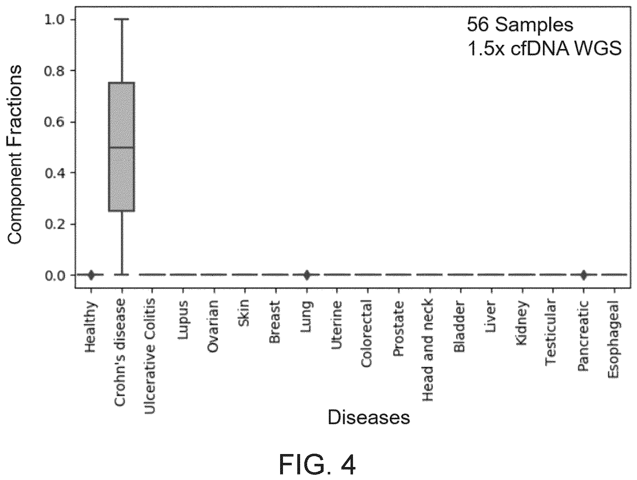

In the field of disease diagnosis and detection, embodiments of the systems, methods, and compositions may be used, for example, for monitoring or analyzing diseases that are hard to diagnose or are frequently misdiagnosed, for example, irritable bowel syndrome, inflammatory bowel disease, celiac disease, fibromyalgia, rheumatoid arthritis, multiple sclerosis, lupus, polycystic ovary syndrome, appendicitis, Crohn's disease, ulcerative colitis, or idiopathic myopathies. Some of these diseases are generally only reliably diagnosed with tissue biopsy. Many diseases are currently diagnosed using tissue biopsy, such as celiac disease. There are many diseases that have no existing diagnosis markers or lack good diagnostic markers, for example, chronic fatigue syndrome. Embodiments of the systems, methods, and compositions provided herein enable monitoring, detecting, evaluating, predicting, or diagnosing of these and other diseases and disorders. For example, embodiments of the systems and methods may be used to determine fractions of a certain tissue component for identifying a certain disease. As shown in Figure 4, for example, a component fraction of colon cfDNA is shown across various diseases, where the fraction for Crohn's disease is significantly greater than in other diseases analyzed.

In the field of oncology, embodiments of the systems, methods, and compositions may be used, for example, for tissue origin quantification of cfDNA and determination of cancer tissue origin as well as the mutations from a single cfDNA whole genome sequence (WGS) assay. A WGS includes the entire sequence (including all chromosomes) of an individual's germline genome.

In the field of NIPT and newborn health monitoring, embodiments of the systems, methods, and compositions may be used, for example, for determining and monitoring maternal health status, and measuring maternal immune reaction towards the fetus. Some embodiments relate to predicting miscarriage and preterm labor. Some embodiments relate to monitoring, investigating, diagnosing, or predicting newborn health conditions, such as organ prematurity, jaundice, genetic defects, or other newborn health conditions, through newborn plasma cfDNA sequencing.

In the field of disease pathology research, embodiments of the systems, methods, and compositions may be used, for example, for simple and low cost tissue-origin-quantification to enable longitudinal studies for researchers to understand pathogenesis of many diseases, by profiling the dynamics and interactions among multiple human organs.

Accordingly, some embodiments provided herein relate to methods and systems for quantification of cfDNA in a subject. In some embodiments, the methods include obtaining a biological sample that is known to carry cfDNA, such as blood plasma, from a subject having or suspected of having a specific type of cancer. As used herein, "cancer" refers to all types of cancer or neoplasm or malignant tumors found in mammals especially humans, including leukemias, sarcomas, carcinomas and melanoma. Examples of cancers are cancer of the brain, breast, cervix, colon, head and neck, kidney, lung, non-small cell lung, melanoma, mesothelioma, ovary, sarcoma, stomach, uterus and medulloblastoma. Additional cancers can include, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, cervical cancer, endometrial cancer, adrenal cortical cancer, and prostate cancer.

In some embodiments, a whole genome sequence (WGS) analysis is performed on the cfDNA in the biological sample to identify regions that may show elevated or decreased quantities of cfDNA compared to quantities of cfDNA in a healthy patient, or compared to cfDNA levels across a cross section of healthy patients. For example, if the patient suffers from liver damage or liver cancer, one may expect to see elevated cfDNA levels identified as being derived from the liver as compared to levels of cfDNA from the liver from a baseline control population. Levels of a certain type of cfDNA may be determined from a total cfDNA level through various algorithms provided herein, including analysis through a variety of machine learning, artificial intelligence, or other algorithms to identify levels and differences of a specific cfDNA from a subject compared to a baseline control, or to identify and compare levels and differences of multiple types of cfDNA derived from multiple tissue types. In some embodiment, analysis of cfDNA includes quantifying the relative fractions of cfDNA from different tissues from the subject and normal baseline controls. In some embodiments, quantification may include one or both of determining the set of reference tissue profiles, and quantifying a fraction of tissue cfDNA in a cfDNA sample based upon a genome-wide cfDNA coverage data. Baseline controls may include healthy control samples from a population of samples, including samples from various geographic regions, ages, ethnicity, race, or gender to establish a proper baseline.

Some embodiments provided herein relate to methods of analyzing cell free DNA (cfDNA) in a biological sample. In some embodiments, the methods include obtaining a biological sample comprising cfDNA; enriching cfDNA from the sample to provide enriched cfDNA, wherein the enriched cfDNA comprises a plurality of cfDNA fragments, each fragment corresponding to a specific tissue or cell type; quantifying each cfDNA fragment to generate a genome-wide cfDNA profile, wherein the genome-wide cfDNA profile comprises a plurality of copy number signals, each copy number signal corresponding to a cfDNA fragment; and comparing the genome-wide cfDNA profile to a profile of known cfDNA copy number signatures to determine cell damage, tissue damage, or organ damage.

In some embodiments, the biological sample may be any biological sample having or suspected of having a profile of cfDNA. Thus, the biological sample may be any sample derived or obtained from a subject, such as a bodily fluid obtained from a subject. Thus, by way of example, a biological sample may be, or may be derived from or obtained from blood, plasma, serum, urine, cerebrospinal fluid, saliva, lymphatic fluid, aqueous humor, vitreous humor, cochlear fluid, tears, milk, sputum, vaginal discharge, or any combination thereof.

In some embodiments, enriching a nucleic acid of interest, or a fragment thereof, such as enriching cfDNA in a sample, may include any suitable enrichment techniques. In some embodiments, enrichment of cfDNA may include enrichment through molecular inversion probes, in solution capture, pulldown probes, bait sets, standard PCR, multiplex PCR, hybrid capture, endonuclease digestion, DNase I hypersensitivity, and selective circularization. Enrichment can be achieved through negative selection of nucleic acids by eliminating undesired material. This sort of enrichment includes 'footprinting' techniques or 'subtractive' hybrid capture. During the former, the target sample is safe from nuclease activity through the protection of protein or by single and double stranded arrangements. During the latter, nucleic acids that bind 'bait' probes are eliminated. In some embodiments, enriching includes amplification of the cfDNA. In some embodiments, amplification comprises PCR amplification or genome-wide amplification.

In some embodiments, quantifying a nucleic acid, such as quantifying cfDNA may include any technique suitable for determining an amount of nucleic acid or nucleic acid fragment in a sample. Thus, for example, quantifying may include sequencing the cfDNA using sequencing-based DNA molecule counting or performing hybridization-based DNA quantification.

In some embodiments, each copy number signal is indicative of a relative contribution of cfDNA from a specific tissue or cell type. A copy number, as used herein, refers to a genome wide cfDNA coverage in a sample, based on signals obtained through DNA molecule counting, such as by any sequencing technologies, or by hybridization-based DNA copy number quantification technologies.

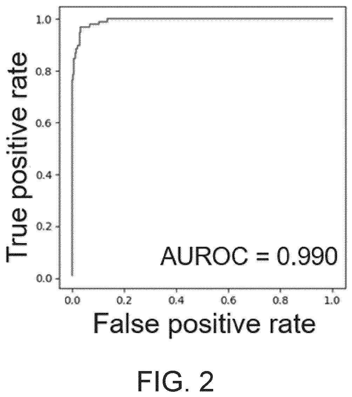

In some embodiments, the tissue type is any tissue type that is desired to be monitored, analyzed, measured, or for which suspected damage is or may be occurring. In some embodiments, the tissue type is kidney, muscle, heart, vascular, liver, brain, eye, lung, adipose, gland, bone, bone marrow, cartilage, intestine, stomach, skin, or bladder. In some embodiments, the cell type is blood cells, neuron cells, kidney cells, epithelial, extracellular matrix cells, or immune cells, or any combinations of cells. For example, the method may include measuring or monitoring one or a plurality of tissue or organ types in a subject. Thus, in some embodiments, the genome-wide cfDNA profile quantifies an amount of cfDNA from multiple organs for providing an assessment of organ health. In some embodiments, each cfDNA fragment is quantified simultaneously. As used herein, simultaneous refers to an action that takes place at the same time or at substantially the same time. Thus, simultaneous quantification refers to analyzing a plurality of cfDNA fragments in a single assay at the same time or substantially at the same time. Accordingly, embodiments provided herein relate to a single analysis universal blood test, wherein multiple organs are or are capable of being monitored in a single assay. For example, quantification of tissue cfDNA may be determined on numerous or a single tissue. One example may be quantification of kidney cfDNA fractions. As shown in Figure 2, kidney fraction is higher for patients with kidney failure, and the quantification described herein enables prediction of kidney failure.

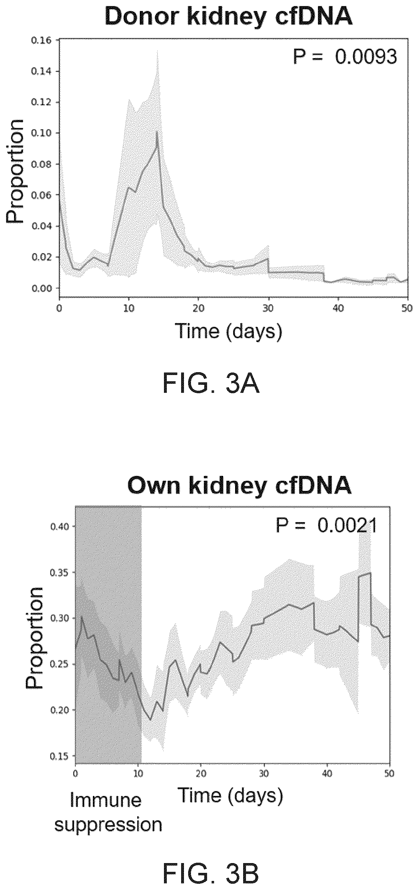

In some embodiments, the sample is obtained and analyzed periodically from a subject to monitor health over time, such that an initial sample is analyzed at a first time point, and a second sample is analyzed at a second time point, and differences in the cfDNA profile are assessed to provide an indication of changes in the cfDNA profile. Such analyses may provide information related to improvement or worsening of certain tissue types over time. For example, such methods may be used to monitor organ transplant, to monitor drug toxicity, to monitor treatment regimens, to monitor health status of various organs or tissues over time, to monitor maternal health during different stages of pregnancy, to monitor newborn health during pregnancy and prior to birth or after birth, or for other suitable assessments. Thus, some embodiments provided herein relate to monitoring organ transplant over time. In some embodiments, the genome-wide cfDNA profile is indicative of drug toxicity in an organ. In some embodiments, the sample is a maternal sample, and the genome-wide cfDNA profile is indicative of fetus health. Suitable periods of time for monitoring a certain tissue, organ, cell, or condition may be dependent on the specific application, and may be on the order of minutes, for example monitoring the sample every 1, 2, 3, 4, 5, 6, 7, 8, 9, 10, 12, 15, 20, 25, 30, 35, 40, 45, 50, 55, or 60 minutes, hours, for example every 1, 2, 3, 4, 5, 6, 7, 8, 9, 10, 12, 15, 18, 20 or 24 hours, days, for example 1, 2, 3, 4, 5, 6, 7, 8, 9, 10, 15, 20, 25, or 30, months, for example, 1, 2, 3, 4, 5, 6, 7, 8, 9, 10, 11, or 12, or years, for example, 1, 2, 3, 4, 5, 6, 7, 8, 9, 10, 15, 20, 25, 30, 35, 40, 45, 50, 55, 60, 65, 70, 75, 80 or more years, or for an amount of time within a range defined by any two of the aforementioned values. For example, a kidney organ transplant may be monitored overtime using the systems and methods described herein. As shown in Figures 3A-3B, time course pattern of the proportion of DNA from kidney tissue as a function of time for donor kidney cfDNA and the patient's own kidney cfDNA may be monitored over time.

In some embodiments, the methods further include subtracting a baseline reference profile from the genome-wide cfDNA profile. A baseline reference profile corresponds to a specific cell or tissue type presented in baseline cfDNA samples, such that the baseline profile may be accounted for in a test sample, and changes or variations from the baseline may be used for diagnostic or abnormality detection.

Some embodiments provided herein relate to methods of monitoring the progress of cancer in a subject. In some embodiments, the methods include obtaining a biological sample from the subject, wherein the biological sample comprises cell free DNA (cfDNA); quantifying the cfDNA in the sample to obtain a genome-wide cfDNA profile comprising a plurality of copy number signals, each copy number signal corresponding to a cfDNA fragment of a specific cell type or tissue type; and comparing the plurality of copy number signals to a profile of known copy number signals of healthy subjects. In some embodiments, a difference of copy number signal in the sample compared to the known copy number signals correlates to a cancerous or precancerous condition in the subject. In some embodiments, total cfDNA is enriched from the sample, prior to quantifying the cfDNA. In some embodiments, the methods further include comparing the plurality of copy number signals to a profile of known copy number signals of cancer patient samples. In some embodiments, the biological sample comprises blood, plasma, serum, urine, cerebrospinal fluid, saliva, lymphatic fluid, aqueous humor, vitreous humor, cochlear fluid, tears, milk, sputum, vaginal discharge, or any combination thereof. In some embodiments, quantifying comprises sequencing the cfDNA using sequencing-based DNA molecule counting. In some embodiments, quantifying comprises performing hybridization-based DNA quantification. In some embodiments, the methods further include enriching cfDNA prior to quantifying the cfDNA. In some embodiments, enriching comprises amplifying the cfDNA through PCR amplification or genome-wide amplification.

The invention includes the following aspects defined in the following clauses which form part of the present description, which, however, do not represent claims, in accordance with the decision J15/88 of the Legal Board of Appeal of the European Patent Office.

1. A method of analyzing cell free DNA (cfDNA) in a biological sample, comprising:

obtaining a biological sample comprising cfDNA;
extracting cfDNA from the sample to provide purified cfDNA, wherein the purified cfDNA comprises a plurality of cfDNA fragments, each fragment corresponding to a specific tissue or cell type;
quantifying the cfDNA fragments to generate a genome-wide cfDNA copy number profile, wherein the genome-wide cfDNA copy number profile comprises a plurality of copy number signals, each copy number signal corresponding to a cfDNA fragment; and
comparing the genome-wide cfDNA copy number profile to a set of known cfDNA signatures to determine cell damage, tissue damage, or organ damage.

2. The method of Clause 1, wherein the biological sample comprises blood, plasma, serum, urine, cerebrospinal fluid, saliva, lymphatic fluid, aqueous humor, vitreous humor, cochlear fluid, tears, milk, sputum, vaginal discharge, or any combination thereof.

3. The method of Clause 1, wherein quantifying comprises sequencing the cfDNA using sequencing-based DNA molecule counting.

4. The method of Clause 1, further comprising enriching cfDNA fragments of interest.

5. The method of Clause 1, wherein quantifying comprises performing hybridization-based DNA quantification.

6. The method of Clause 1, wherein extracting comprises size-based enrichment to exclude gDNA and enrich for cfDNA fragments.

7. The method of Clause 1, wherein enriching comprises amplification of the cfDNA.

8. The method of Clause 7, wherein amplification comprises PCR amplification or genome-wide amplification.

9. The method of Clause 1, wherein each copy number signal is indicative of a relative contribution of cfDNA from a specific tissue or cell type.

10. The method of Clause 1, wherein the tissue type is kidney, muscle, heart, vascular, liver, brain, eye, lung, adipose, gland, bone, bone marrow, cartilage, intestine, stomach, skin, or bladder.

11. The method of Clause 1, wherein the cell type is blood cells, neuron cells, kidney cells, epithelial, extracellular matrix cells, beta cells, or immune cells.

12. The method of Clause 1, wherein the genome-wide cfDNA profile is used to quantify amounts of cfDNA from multiple organs for providing an assessment of organ or multi-organ health.

13. The method of Clause 1, wherein the sample is obtained and analyzed periodically from a subject to monitor health over time.

14. The method of Clause 13, further comprising monitoring organ transplant over time.

15. The method of Clause 1, wherein the genome-wide cfDNA profile is indicative of drug toxicity or efficacy in an organ.

16. The method of Clause 1, wherein the sample is a maternal sample, and wherein the genome-wide cfDNA profile is indicative of fetus health.

17. The method of Clause 1, wherein multiple organs are quantified simultaneously by projecting the genome-wide cfDNA profile onto a set of reference cfDNA profiles corresponding to various tissue and cell types.

18. The method of Clause 1, further comprising subtracting a baseline reference profile from the genome-wide cfDNA profile.

19. A method of monitoring disease progress in a subject, comprising:

obtaining a biological sample from the subject, wherein the biological sample comprises cell free DNA (cfDNA); quantifying the cfDNA in the sample to obtain a genome-wide cfDNA profile comprising a plurality of copy number signals, each copy number signal corresponding to a cfDNA fragment of a specific cell type or tissue type; and comparing the plurality of cfDNA copy number signals to a set of known copy number signals of healthy subjects, wherein a difference of copy number signal in the sample compared to the known copy number signals correlates to a disease progress in the subject.

20. The method of Clause 19, wherein total cfDNA is enriched from the sample, prior to quantifying the cfDNA.

21. The method of Clause 19, further comprising comparing the plurality of copy number signals to a profile of known copy number signals of diseased patient samples.

22. The method of Clause 19, wherein the biological sample comprises blood, plasma, serum, urine, cerebrospinal fluid, saliva, lymphatic fluid, aqueous humor, vitreous humor, cochlear fluid, tears, milk, sputum, vaginal discharge, or any combination thereof.

23. The method of Clause 19, wherein quantifying comprises sequencing the cfDNA using sequencing-based DNA molecule counting.

24. The method of Clause 19, wherein quantifying comprises performing hybridization-based DNA quantification.

25. The method of Clause 19, wherein the disease is selected from heart failure, lung damage, diabetes, Crohn's disease, or kidney disease.

26. The method of Clause 25, wherein enriching comprises amplifying the cfDNA through targeted amplification or genome-wide amplification.

27. A method of monitoring tissue and organ health in a subject, comprising:

obtaining a biological sample from the subject, wherein the biological sample comprises cell free DNA (cfDNA); quantifying the cfDNA in the sample to obtain a genome-wide cfDNA profile comprising a plurality of copy number signals, each copy number signal corresponding to a cfDNA fragment of a specific cell type or tissue type; and comparing the plurality of cfDNA copy number signals to a set of known copy number signals of healthy subjects, wherein a difference of copy number signal in the sample compared to the known copy number signals correlates to a change in organ health condition in the subject.

28. The method of Clause 27, wherein total cfDNA is enriched from the sample, prior to quantifying the cfDNA.

29. The method of Clause 27, further comprising comparing the plurality of copy number signals to a profile of known copy number signals of samples from a patient having poor tissue or organ health.

30. The method of Clause 27, wherein the biological sample comprises blood, plasma, serum, urine, cerebrospinal fluid, saliva, lymphatic fluid, aqueous humor, vitreous humor, cochlear fluid, tears, milk, sputum, vaginal discharge, or any combination thereof.

31. The method of Clause 27, wherein quantifying comprises sequencing the cfDNA using sequencing-based DNA molecule counting.

32. The method of Clause 27, wherein quantifying comprises performing hybridization-based DNA quantification.

33. The method of Clause 27, wherein enriching comprises amplifying the cfDNA through targeted amplification or genome-wide amplification.

EXAMPLES

Additional alternatives are disclosed in further detail in the following examples, which are not in any way intended to limit the scope of the claims.

## General Procedures and Methods

### Extraction

Normal blood circulation rate is about 5 liters per minute, such that the full volume of blood circulates once per minute. This rate is far higher than cfDNA generation and degradation kinetics, and cfDNA composition is uniform in a person's blood within a short time frame (e.g. less than 5 minutes). Under these conditions, a blood draw is approximately a Poisson sampling of cfDNA. Either a multinomial distribution or a multivariate hypergeometric distribution is used to model the DNA extraction.

The extraction process follows a Poisson distribution $n''_l \sim \text{Pois}(n'' \cdot \Sigma_t \beta_t \cdot A_{tl})$, or jointly a multinomial distribution $(n''_l) \sim \text{Multi}(\Sigma_t \beta_t \cdot A_t, n'')$, where $n''_l$ is the copy numbers at locus $l$, $n''$ is the total copies of cfDNA fragments, $\beta_t$ is the fraction of cfDNA from tissue type t, and $A_t$ is the reference copy number profile for tissue type t.

### PCR Amplification

The PCR process is approximated by a Gamma distribution $n'_l \sim \text{Gamma}(n''_l \cdot p, \theta)$, or jointly a Dirichlet distribution $(n'_l)/\theta \sim \text{Dir}(\alpha = (n''_l \cdot \rho))$, where $\rho = (1+r)/(1-r)/[1-(1+r)^{-t}]$, $\theta = [(1+r)^t - 1] \cdot (1-r)/(1+r)$, and r is PCR amplification efficiency in each cycle, , $n'_l$ is the number of DNA molecules at locus $l$ after PCR, $n'$ is the total number of DNA molecules amplified from cfDNA fragments.

### Sequencing

Similar to extraction, sequencing follows a Poisson distribution $n_l \sim \text{Pois}(n \cdot n'_l / n')$, or jointly a multinomial distribution $(n_l) \sim \text{Multi}(n'_l/n', n)$, where n is the number of fragments observed in sequencing, and $n_l$ is the observed cfDNA copy number at a given locus $l$.

### Some Number

**[0065]** With approximately 5,000 mL of blood in a typical person, 1.8-44 ng/mL plasma cfDNA corresponds to 1.35-33 million copies of human genomes. A tissue fraction of 1% corresponds to 13,500-330,000 copies. By way of example, where 3 ng of cfDNA is used as input for a cfDNA WGS assay, this corresponds to 900 copies total, 9 copies of a 1% tissue genome, and 0.9 copies of a 0.1% tissue genome.

### Example 1 - Modeling an Aggregated cfDNA Signal Profile

The following example demonstrates an embodiment of modeling an aggregated cfDNA signal profile.

Ignoring extraction and PCR variabilities, the model S of cfDNA signal is $(n_l) \sim \text{Multi}(\Sigma_t \beta_t \cdot A_t, n)$. Given a large number of bins (or loci) that are approximately evenly distributed, it is close to a Poisson distribution: $n_l \sim \text{Pois}(n \cdot \Sigma_t \beta_t \cdot At)$. Given known tissue profiles A, only unknowns are the tissue fractions $B = (\beta_t)$, which can be solved by numerical optimization.

Model PS of cfDNA signal is a gamma-Poisson (negative binomial) distribution $n_l \sim \text{NB}(n''_l \cdot p, p = n \cdot \theta / (n' + n \cdot \theta))$. Given $n' = n'' \cdot \rho \cdot \theta$, $n''_l = n'' \cdot \Sigma_t \beta_t \cdot A_{tl}$, and ignoring the variability from extraction gives $n_l \sim \text{NB}(n'' \cdot \rho \cdot \Sigma_t \beta_t \cdot A_{tl}, n/(n'' \cdot \rho + n))$. When $n \ll n'' \cdot \rho$, it is approximately $n_l \sim \text{Pois}(n \cdot \Sigma_t \beta_t \cdot A_{tl})$, which is the same as model S.

Combining E and P steps into a single Dirichlet distribution $(n'_l)/\theta \sim \text{Dir}(n'' \cdot \alpha \cdot 1/(1+1/p))$, or $n'_l \sim \text{Gamma}(n'' \cdot \alpha \cdot \rho/(1+\rho), (1+\rho) \theta)$. The Dirichlet Distribution is used to estimate an unknown multinomial probability distribution. More specifically, it extends Beta distribution into multiple dimensions and provides a smooth transition between the prior distribution and the observed distribution and allows for control over how quickly that transition occurs.

Combining extraction, PCR, and sequencing step together, the model EPS of cfDNA signal is $(n_l) \sim \text{DM}(n'' / (1+1/p) \cdot \alpha, n)$ or $(n_l) \sim \text{DM}(n'' \cdot \alpha \cdot (1+r)/2, n)$, where DM is a Dirichlet-Multinomial distribution. Given a large number of bins (or loci) that are approximately evenly distributed, it is close to an negative binomial distribution: $n_l \sim \text{NB}(n'' \cdot \alpha \cdot \rho/(1+\rho), (1+\rho)\theta n / [(1+\rho)\theta n + n'])$ or $n_l \sim \text{NB}(n'' \cdot \alpha_l \cdot (1+r)/2, n/[n + n'' \cdot (1+r)/2])$. The mean and variance of the $\mu = n \cdot \alpha_l$, $\delta^2 = n \cdot \alpha_l \cdot [n/n'' \cdot (1/\rho + 1) + 1]$. When $n \ll n''$, for example, for 30x WGS with > 1 ng input cfDNA, $n_l$ approaches Poisson distribution $n_l \sim \text{Pois}(n \cdot \alpha_l)$. Table 1 provides a list of probabilistic models involved in cfDNA quantification, where $\alpha_l = \Sigma_t \beta_t \cdot A_{tl}$, and $\alpha = \Sigma_t \beta_t \cdot$

$A_t$.

Table 1

|  | Dependent Model | Independent Model |
|---|---|---|
| Component E | $(n"_l) \sim \text{Multi}(\alpha, n")$ | $n"_l \sim \text{Pois}(n" \cdot \alpha_l)$ |
| Component P | $(n'_l) / \theta \sim \text{Dir}((n"_l \cdot \rho))$ | $n'_l \sim \text{Gamma}(n"_l \cdot \rho, \theta)$ |
| Component S | $(n_l) \sim \text{Multi}( (n'_l / n') , n)$ | $n_l \sim \text{Pois}(n \cdot n'_l / n')$ |
| Model S | $(n_l) \sim \text{Multi}(\alpha, n)$ | $n_l \sim \text{Pois}(n \cdot \alpha_l)$ |
| Model PS | $(n_l)) \sim \text{DM}(n" \cdot \rho \cdot \alpha, n)$ | $n_l \sim \text{NB}(n" \cdot \rho \cdot \alpha_l, n/(n" \cdot \rho + n))$, or $n_l \sim \text{Pois}(n \cdot \alpha_l)$, if $n << n" \cdot \rho$. |
| Model EPS | $(n_l) \sim \text{DM}(n"/(1+1/\rho) \cdot \alpha, n)$, | $n_l \sim \text{NB}(n" \cdot a_l \cdot \rho/(1+\rho), n/[n + n" \cdot \rho/(1+\rho)]$, or $n_l \sim \text{Pois} (n \cdot \alpha_l)$, if $n << n"$. |

Model PS of cfDNA signal is a gamma-Poisson (negative binomial) distribution $n_l \sim \text{NB}(n"_l \cdot \rho, \rho = n \cdot \theta / (n' + n \cdot \theta))$. Given $n' = n" \cdot \rho \cdot \theta$, $n"_l = n" \cdot \Sigma_t \beta_t \cdot A_{tl}$, and ignoring the variability from extraction gives $n_l \sim \text{NB}(n" \cdot \rho \cdot \Sigma_t \beta_t \cdot A_{tl}, n / (n" \cdot \rho + n))$. When $n << n" \cdot \rho$, it is approximately $n_l \sim \text{Pois}(n \cdot \Sigma_t \beta_t \cdot A_{tl})$, which is the same as model S.

## Multiplicative Updating

The Poisson model $n_l \sim \text{Pois}(n \cdot \alpha_l)$ is equivalent to Non-negative matrix factorization with KL divergence as cost. Applying the multiplicative updating algorithm $\beta_{st} \leftarrow \beta_{st} \cdot \Sigma_l A_{tl} \cdot r_{sl} / (\beta \bullet A)_{sl} / \Sigma_l A_{tl}$ based on the non-negative matrix factorization (NMF) algorithm described in Lee and Seung, 2001, is used to compute $\beta_t$.

## Iterative Weighted Linear Regression

For a given sample, with an estimated tissue fraction $\beta_0$, a weighted linear regression with cost function is defined as $E(\beta; \beta_0, A) = 1/2 \cdot \Sigma_l [(r_l - (\beta \bullet A)_l)^2 / (\beta_0 \bullet A)_l]$. This weighted linear regress is solved $(\beta_0, A)$, then $\beta \leftarrow r \bullet W^{-1} \bullet A^T (A \bullet W^{-1} \bullet A^T)^{-1}$, where $W = \text{diag}(\beta_0 \bullet A)$, providing a further iterative updating algorithm. The difference between this and regular linear regression $E = 1/2 \cdot \Sigma_l [(r_l - (\beta \bullet A)_l)^2$ is a weighting based on $W = \text{diag}(\alpha) = \beta \bullet A_L$.

## Derivation of Model EPS

Given $(n'_l) / \theta \sim \text{Dir}((n"_l \cdot \rho))$ and $(n"_l) \sim \text{Multi}(\alpha, n")$, and the law of total variance is given as:

$$E((n'_l) / \theta) = \alpha,$$

$$\text{var}((n'_l) / \theta) = \text{var}(n"_l / n") + E(n"_l \cdot \rho ( n" \cdot \rho - n"_l \cdot \rho)/ [(n" \cdot \rho)^2(n" \cdot \rho + 1)].$$

$$\sim= \text{var}(n"_l / n") + E(n"_l / n" (1 - n"_l / n")/ [n" \cdot \rho]).$$

$$= \alpha (1-\alpha) / n" + \alpha / [n" \cdot \rho] - (\text{var}(n"_l / n" ) + \alpha^2) / [n" \cdot \rho])$$

$$= \alpha (1-\alpha) / n" + \alpha / [n" \cdot \rho] - (\alpha (1-\alpha) / n" + \alpha^2) / [n" \cdot \rho])$$

$$= \alpha (1-\alpha) \{1 / n" (1 - 1/[n" \cdot \rho]) + 1/ [n" \cdot \rho]\}$$

$$\sim= \alpha (1-\alpha) \{1 / n" + 1/ [n" \cdot \rho]\}$$

$$= \alpha (1-\alpha) / [n" \cdot 1/(1+ 1/\rho))]$$

This matches a $\text{Dir}(n" \cdot \alpha \cdot 1/(1+ 1/p))$. Given $n"_l \sim \text{Pois}(n" \cdot \alpha_l)$ and $n'_l \sim \text{Gamma}(n"_l \cdot \rho, \theta)$, and the law of total variance gives:

$$E((n'_l)) = n'' \cdot \alpha_l \cdot \rho \cdot \theta,$$

$$var((n'_l)) = var(n''_l \cdot \rho \cdot \theta) + E(n''_l \cdot \rho \; \theta^2)$$
$$= n'' \cdot \alpha_l \cdot \rho \; (1+\rho) \; \theta^2$$

This matches a Gamma$(n'' \cdot \alpha \cdot \rho/(1+\rho), (1+\rho) \theta)$.

$$n \cdot n'_l / n' \sim Gamma(n'' \cdot \alpha \cdot \rho/(1+\rho), (1+\rho) \; \theta \; n / n')$$

$$n_l \sim Pois(n \cdot n'_l / n')$$

$$n_l \sim NB(n'' \cdot \alpha \cdot \rho/(1+\rho), (1+\rho) \; \theta \; n / [(1+\rho) \; \theta \; n + n'])$$

$$n_l \sim NB(n'' \cdot \alpha \cdot \rho/(1+\rho), (1+\rho) \; n / [(1+\rho) \; n + n'' \cdot \rho])$$

Example 2 - Determining Tissue cfDNA Profile

The following example demonstrates embodiments of a method for determining a tissue cfDNA reference profile.
Two complementary strategies may be used for estimating tissue specific or cell type specific cfDNA signal profiles. The first method is to use unsupervised machine learning, based on a set of samples that contain the tissue/cell of interest at varying fractions. The second method is to use supervised machine learning, by predicting the cfDNA signal profiles originated from a given tissue/cell based on the genomic DNA (gDNA) epigenetic profiles or gene expression profiles of the tissue/cell type.

**Unsupervised Machine Learning**

The supervised machine learning method applies non-negative matrix factorization to decompose cfDNA mixture signal and extract the tissue specific cfDNA coverage profiles. The Poisson model $n_l \sim Pois(n \cdot \alpha_l)$ is equivalent to non-negative matrix factorization with a Kullback-Leibler (KL) divergence as cost. A KL divergence is a measure of how one probability distribution differs from a reference probability distribution. For a given dataset of sufficient size and tissue composition of a tissue type of interest, the NMF algorithm by Lee and Seung 2001 is applied to estimate tissue fractions in each sample, as well as to ascertain the tissue cfDNA profiles. Tissue fraction for tissue t in sample s is estimated by $\beta_{st} \leftarrow \beta_{st} \cdot \Sigma_l A_{tl} \cdot r_{sl} / (\beta \bullet A)_{sl} / \Sigma_l A_{tl}$, whereas cfDNA signal at locus l for tissue type t is estimated by $A_{tl} \leftarrow A_{tl} \cdot \Sigma_s \beta_{st} \cdot r_{sl} / (\beta \bullet A)_{sl} / \Sigma_s \beta_{st}$, where • is matrix multiplication, $r_{sl}$ is the fraction of reads covering locus l in sample s.

**Supervised Machine Learning**

There are two related limitations of the unsupervised algorithm. First it requires samples from individuals under specific physiological or disease conditions, for example, to learn kidney cfDNA profile, access to multiple cfDNA samples from patients with elevated kidney damage is required. Second, for tissue types with small cell populations or cell type that is rare, the fraction of blood cfDNA signal contributed by such cells could be very small. Thus, a larger number of cfDNA samples is required to effectively learn the cfDNA signal profiles for such tissue or cell types. These limitations may be overcome by large datasets. However, in practice, large datasets may prevent the wide application cfDNA WGS-based tissue quantification to all tissue types.
For these reasons, supervised machine learning that predicts tissue specific cfDNA copy number profiles from epigenetic or expression data from the specific tissue cell samples may be used. Supervised machine learning does not require access to cfDNA samples from patients with specific organ damage, but instead only uses isolated tissue cells from either normal or disease samples. The methods apply deep neural network, and more specifically recurrent neural network or convolutional neural network on one-dimensional sequencing data, to predict cfDNA profiles. The input features to the neural networks include genome wide DNase accessibility, DNA methylation, histone methylation, histone acetylation profiles, or gene expression profiles for the given tissue type. The prediction from the machine learning is a genome wide cfDNA copy number profile for the tissue of interest.
Both within-tissue and cross-tissue cross-validation is used to train and evaluate the machine learning models.

More specifically, tissue specific epigenetic data are prepared as input feature, and estimated tissue cfDNA coverage profiles (from the unsupervised algorithms) are prepared as target. For within-tissue cross-validation, a subset of loci in the genome for validation is retained, and the other loci is used for training. For cross-tissue cross-validation, cfDNA reference profiles for certain cell types, such as blood cells, are used for training, and cfDNA reference profiles for additional cell types, such as kidney or lung cells, are used for validation.

Example 3 - cfDNA Studies

The following example demonstrates embodiments of studies for analyzing cfDNA in a sample from subject.

**Pilot Study**

Plasma DNA from 10 patients with end stage renal disease (ESRD) and 10 age-, gender-, and body weight-matched normal controls were obtained and studied. For each sample, 30X WGS was performed. The presence of strong cfDNA signals that can reliably differentiate ESRD vs normal controls were obtained. Clustering analysis and principal component analysis (PCA) show that the ESRD and normal samples form distinct groups. For normal controls, the determined kidney fractions were < 0.5%.

**Mixture Study**

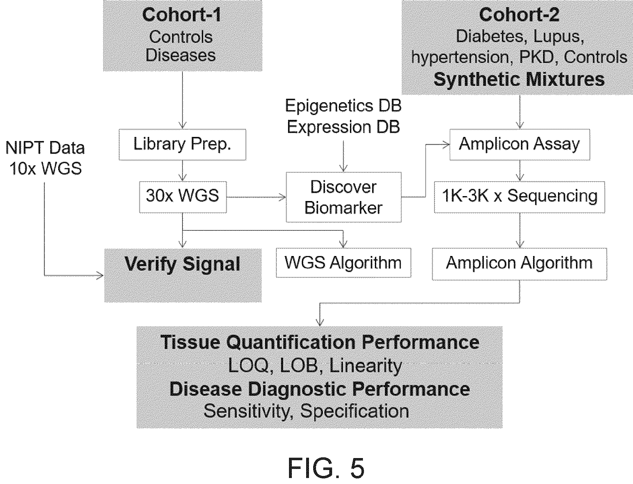

For three case-control pairs, synthetic cfDNA mixtures were prepared by mixing the ESRD with control cfDNA through serial dilutions. For each case-control pair, eight mixtures with 100%, 50%, 25%, 12.5%, 6.25%, 3.125%, 1.5625%, and 0.78125% ESRD cfDNA were diluted with control cfDNA. With this dataset, tissue quantification analytical performance was determined. The mixture study demonstrated that the estimated kidney fraction is linear to the true kidney fraction, and that the kidney fraction can be precisely (CV < 20%) determined for as low as 0.5%.
One embodiment for validation is depicted in the block diagram of Figure 5, which illustrates a process for evaluating cfDNA samples for tissue cfDNA quantification. As shown in Figure 5, a first cohort may include control and diseased subjects, which is subjected to library preparation, 30x WGS, and then analyzed. Portions of the WGS product are subjected to biomarker discovery, whereas other portions are subjected to signal verification or WGS algorithms. A second cohort may be a cohort of synthetic mixtures, including, for example, numerous samples from diabetes subjects, lupus subjects, hypertension subjects, kidney disease (such as chronic kidney disease (CKD) or polycystic kidney disease (PKD)), control samples, or samples from other subjects. The mixtures are applied to an amplicon assay, sequencing, and algorithms to determine the performance of the methods for quantifying tissue (including a determination of a limit of quantification (LOQ) or limit of detection LOD) and linearity of the methods) or diagnosing disease (including determination of the sensitivity and specification of the methods.

**Full Study**

Following the mixture study, around 200 diabetic patient samples at various stages of chronic kidney disease (CKD) are collected and subjected to 30x cfDNA WGS. The results indicate that the estimated kidney fraction can reliably differentiate patients with early stage CKD versus end stage CKD, that the estimated kidney fraction can reliably differentiate patients with early stage CKD versus diabetic patients without CKD, and that the estimated kidney fraction is correlated with the severity of kidney disease.

**Diverse Organ Study**

Five blood samples from patients with heart failure or lung damage (e.g., cystic fibrosis) or normal controls are collected and subject to 30x cfDNA WGS. The results demonstrate that patients with heart failure, lung damage, or kidney disease have distinct cfDNA signal profiles among each other, and they are different from normal controls, and that heart cfDNA fractions and lung cfDNA fractions can be quantified.

**Diverse Transplant Study**

Five blood samples from patients with lung or heart transplants are collected and subject to 30x cfDNA WGS. The results demonstrate that patients with heart transplants or lung transplants have distinct patterns, and that estimated lung fractions or heart fractions are linearly correlated to genetic variant-based donor organ fractions.
The term "comprising" as used herein is synonymous with "including," "containing," or "characterized by," and is inclusive or open-ended and does not exclude additional, unrecited elements or method steps.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope and spirit of the invention.

All references cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification, the specification is intended to supersede and/or take precedence over any such contradictory material.

**Claims**

1. A method of analyzing cell free DNA (cfDNA) in a biological sample, comprising:

   extracting cfDNA from a first biological sample to provide purified cfDNA, wherein the purified cfDNA comprises a plurality of cfDNA fragments, each fragment corresponding to a specific tissue or cell type;
   inputting to one or more hardware processors implementing a supervised machine learning model, one or more of: a genome wide DNase accessibility, a DNA methylation, a histone methylation, histone acetylation profiles, or gene expression profiles for a target tissue type, wherein the supervised machine learning model was trained on data from isolated tissue cells of the target tissue type, the isolated tissue cells originating from a normal sample or a disease sample;
   generating a genome-wide cfDNA copy number profile based on the first biological sample using the one or more hardware processors implementing the supervised machine learning model; and
   determining, based on the genome-wide cfDNA copy number profile, tissue health corresponding to the target tissue type.

2. The method of Claim 1, wherein the biological sample comprises blood, plasma, serum, urine, cerebrospinal fluid, saliva, lymphatic fluid, aqueous humor, vitreous humor, cochlear fluid, tears, milk, sputum, vaginal discharge, or any combination thereof, optionally, wherein the target tissue type is kidney, muscle, heart, vascular, liver, brain, eye, lung, adipose, gland, bone, bone marrow, cartilage, intestine, stomach, skin, or bladder; and optionally, wherein the cell type is blood cells, neuron cells, kidney cells, epithelial cell, extracellular matrix cells, beta cells, or immune cells.

3. The method of Claim 1, wherein the supervised machine learning model comprises a deep neural network.

4. The method of Claim 3, wherein the deep neural network is a recurrent neural network or a convolutional neural network.

5. The method of Claim 1, further comprising enriching cfDNA fragments of interest, optionally wherein enriching comprises amplification of the cfDNA, optionally wherein amplification comprises PCR amplification or genome-wide amplification.

6. The method of Claim 1, comprising predicting kidney failure based on the determination of the tissue health, wherein the target tissue type is kidney.

7. The method of Claim 1, wherein the target tissue type is a donor tissue.

8. The method of Claim 7, wherein the donor tissue is a donor kidney.

9. The method of Claim 7, comprising comparing a cfDNA signal of the donor tissue to a cfDNA signal of a patient's own tissue.

10. The method of Claim 1, comprising

    extracting cfDNA from a second biological sample obtained at a time different from the first biological sample;
    generating a genome-wide cfDNA copy number profile based on the second biological sample using the one or more hardware processors implementing the supervised machine learning model; and

determining, based on a comparison of the genome-wide cfDNA copy number profile of the first biological sample to the genome-wide cfDNA copy number profile of the second biological sample, a tissue health progression.

11. The method of Claim 10, wherein the second biological sample is obtained from 1 minute to 80 years after the first biological sample,

preferably wherein the second biological sample is obtained about a day after the first biological sample is obtained, or
preferably wherein the second biological sample is obtained about a week after the first biological sample is obtained, or
preferably wherein the second biological sample is obtained about a month after the first biological sample is obtained, or
preferably wherein the second biological sample is obtained about a year after the first biological sample is obtained.

12. The method of Claim 1, comprising

extracting cfDNA from a plurality of biological samples, the plurality of biological samples comprising the first biological sample, wherein each of the plurality of biological samples obtained at different times;
generating a genome-wide cfDNA copy number profile based on each of the plurality of biological samples using the one or more hardware processors implementing the supervised machine learning model; and
determining, based on a comparison of the genome-wide cfDNA copy number profile of each of the genome-wide cfDNA copy number profiles, a tissue health progression.

13. The method of Claim 10, wherein the plurality of biological samples are obtained approximately hourly, or

wherein the plurality of biological samples are obtained approximately daily, or
wherein the plurality of biological samples are obtained approximately weekly, or
wherein the plurality of biological samples are obtained approximately monthly, or
wherein the plurality of biological samples are obtained approximately yearly.

14. The method of Claim 1, comprising monitoring a disease of the tissue type, wherein the disease is irritable bowel syndrome, inflammatory bowel disease, celiac disease, fibromyalgia, rheumatoid arthritis, multiple sclerosis, lupus, polycystic ovary syndrome, appendicitis, Crohn's disease, ulcerative colitis, lupus, ovarian disease, skin disease, breast disease, lung disease, uterine disease, colorectal disease, prostate disease, bladder disease, liver disease, kidney disease, testicular disease, pancreatic disease, or esophageal disease.

15. The method of Claim 14, wherein the monitoring the disease of the tissue type comprises comparing the genome-wide copy number profile to a genome-wide copy number profile of a healthy baseline.

FIG. 1

FIG. 2

## Donor kidney cfDNA

P = 0.0093

FIG. 3A

## Own kidney cfDNA

P = 0.0021

Immune suppression

Time (days)

FIG. 3B

FIG. 4

FIG. 5

**REFERENCES CITED IN THE DESCRIPTION**

**Non-patent literature cited in the description**

- **SAMBROOK et al.** Molecular Cloning: A Laboratory Manual. Cold Spring Harbor, 2001

- **AUSUBEL et al.** *Current Protocols in Molecular Biology*, 1987